## Europäisches Patentamt

## European Patent Office

## Office européen des brevets

(19)

(11) Veröffentlichungsnummer: **0 103 783**
**A2**

(12) # EUROPÄISCHE PATENTANMELDUNG

(21) Anmeldenummer: 83108436.3

(22) Anmeldetag: 26.08.83

(51) Int. Cl.³: **A 61 K 9/08**
**A 61 K 47/00**

(30) Priorität: 10.09.82 DE 3233638

(43) Veröffentlichungstag der Anmeldung:
28.03.84 Patentblatt 84/13

(84) Benannte Vertragsstaaten:
AT BE CH FR GB IT LI LU NL SE

(71) Anmelder: Dr. Thilo & Co. GmbH
Rudolf-Diesel-Ring 21
D-8029 Sauerlach(DE)

(72) Erfinder: Kreiner, Christine F., Dr.
Krenkistrasse 10
D-8000 München 81(DE) –

(72) Erfinder: Löbering, Hans Georg, Dr. rer.nat.
Vogelherd 10
D-8035 Gauting-Buchendorf(DE)

(74) Vertreter: von Füner, Alexander, Dr. et al,
Patentanwälte Schiff, von Füner Strehl, Schübel-Hopf,
Ebbinghaus, Finck Mariahilfplatz 2 u. 3
D-8000 München 90(DE)

(54) Dermatologische Zubereitung.

(57) Gegenstand der Erfindung sind topisch anzuwendende galenische dünnflüssige bis gallertartige klare Lösungen auf der Basis von kurzkettigen Alkoholen und gegebenenfalls Wasser für die dermatologische Anwendung, wobei erfindungsgemäß längerkettige lipophile Alkohole und gegebenenfalls Fettsäureester und/oder deren Derivate zum Einsatz gelangen. Diese Lösungen gestatten es, in Kombination mit geeigneten Wirkstoffen, insbesondere Virustatika, die Penetration der Wirkstoffe zu steigern.

EP 0 103 783 A2

v. FÜNER  EHBINGHAUS  0103783 K
PATENTANWÄLTE  EUROPEAN PATENT ATTORNEYS
MARIAHILFPLATZ 2 & 3, MÜNCHEN 90
POSTADRESSE: POSTFACH 95 01 60, D-8000 MÜNCHEN 95

Dr. Thilo & Co. GmbH

EPAA-31326.8

26. August 1983

## DERMATOLOGISCHE ZUBEREITUNG

Die vorliegende Erfindung betrifft neuartige, galenische Zubereitungen, die eine wesentlich bessere Bioverfügbarkeit (Targetkonzentration) der Wirkstoffe für eine dermatologische Anwendung ermöglichen.

Viele Wirkstoffe, die in der Dermatologie zur Anwendung gelangen, zeichnen sich durch eine sehr schwach ausgeprägte bzw. fehlende Lipophilie aus. Dies führt zwangsläufig zu einer schlechten dermalen Penetration dieser Substanzen aus üblichen galenischen Grundlagen (Salben, Cremes, Gele, wäßrige Lösungen), so daß viele dieser Zubereitungen nur eine unbefriedigende oder möglicherweise keine Wirksamkeit bei dermatologischer Anwendung aufweisen. Die geringe Lipophilie der Wirkstoffe bedingten neben einer sehr geringen Lipidlöslichkeit, eine zum Teil recht gute Wasserlöslichkeit. Ein solcher Wirkstoff liegt normalerweise in den üblichen galenischen Zubereitungen entweder in den Wasserbestandteilen gelöst, oder in der gesamten Grundlage suspendiert vor. In beiden Fällen ist die Freisetzung in das Stratum corneum nur mangelhaft.

Um die Penetration der Wirkstoffe zu verbessern, wurden Gleitschienen in Form von oberflächenaktiven Verbindungen oder DMSO eingesetzt. Insbesondere der Zusatz von von DMSO führt tatsächlich zu einer Penetrationsverbesserung (W. Reichert, Z. Hautkr. 55, 1980, S. 773 bis 781), jedoch weisen solche Zusätze eine relativ hohe Toxizität auf. Eine andere, schon lange bekannte Möglichkeit, die Wirksamkeit topisch zu applizierender Substanz zu erhöhen, besteht darin, Tinkturen auf Alkoholbasis herzustellen. Zur längeren Verweildauer der Wirkstoffe auf der Haut wurden zudem Glycerin und/oder Propandiolzusätze eingeführt. Solche Tinkturen bewirken jedoch keine verstärkte Freisetzung des Arzneimittels, sondern sind nur durch eine Hyperämisierung der Hautgefäße gekennzeichnet.

Aufgabe der Erfindung war die Entwicklung einer physiologisch verträglichen galenischen Grundlage mit optimaler Wirkstoff-Freisetzung, um auch bisher topisch unwirksame oder schlecht wirksame Wirkstoffe erfolgreich in der Dermatologie einsetzen zu können. Diese Aufgabe wurde wie aus den vorstehenden Ansprüchen ersichtlich gelöst.

Man stellte also fest, daß Formulierungen aus Alkohol, bzw. wäßrigem Alkohol und 1 bis etwa 50 % öligem Anteil in Form von Fettalkohol, gegebenenfalls Fettsäureestern und Fettsäureesterderivaten, in denen der Wirkstoff in gelöster Form vorliegt, überraschenderweise zu einer erheblichen Wirksamkeitssteigerung führten. Die Zubereitungen, die in Form einer Lösunge (Tinktur), eines Spray oder auch nach Zusatz geeigneter Celluloseätherverbindungen in Form eines Gels auf die Haut aufgebracht werden können, ändern sich auf der Haut durch das schnelle Abdunsten des Alkohols bzw. der wäßrig alkoholischen Komponenten zu einem dauerhaften und aufgrund der geringen Lipidlöslichkeit der Wirkstoffe wirkstoffübersättigten Ölfilm (Öl-

pflaster). Dies führt zu einer Anreicherung der Wirksubstanz im Stratum corneum, das bei der intakten Haut die wesentliche Penetrationsbarriere darstellt. Da bei der intakten Haut lediglich die Konzentration des Wirkstoffs im Stratum corneum entscheidend für die weitere Penetration des Wirkstoffes in die tieferen Schichten ist, bewirkt somit der wirkstoffübersättigte Ölfilm letztlich eine höhere Wirksamkeit der Substanz. Bei verletzter Haut ist lediglich die Wirkstofffreisetzung direkt aus der Grundlage der geschwindigkeitsbestimmende Schritt für die Penetration. Auch hier wirkt sich der wirkstoffübersättigte Ölfilm in der Weise aus, daß eine vermehrte Freisetzung der gut wasserlöslichen Substanz erfolgt.

Die erfindungsgemäßen, topisch anzuwendenden galenischen Zubereitungen sind dünnflüssige bis gallertartige klare Lösungen auf der Basis von kurzkettigen Alkoholen die gegebenenfalls Wasser enthalten können. Erfindungsgemäß enthalten sie längerkettige lipophile Alkohole und gegebenenfalls Fettsäureester bzw. deren Derivate.

Bei den längerkettigen lipophilen Alkoholen handelt es sich um gesättigte oder ungesättigte Fettalkohole der Kettenlänge $C_{12}$ bis $C_{21}$ , wobei Ricinoleylalkohol besonders bevorzugt ist. Beispiele für die erfindungsgemäß verwendeten längerkettigen lipophilen Alkohole sind:

$$n - C_{12}H_{25}OH \qquad \text{Laurylalkohol}$$

$$n - C_{14}H_{29}OH \qquad \text{Myristylalkohol}$$

$$n - C_{16}H_{33}OH \qquad \text{Stearylalkohol}$$

$$C_{18}H_{35}OH \qquad \text{Oleylalkohol (synth.)}$$

bevorzugt $C_{18}H_{35}OH \qquad$ Ricinoleylalkohol (pflanzl. Ursprungs)

Die längerkettigen lipophilen Fettalkohole sollen vorzugsweise in einer Konzentration von 1 bis 50 Vol.-% vorliegen.

Es wurde festgestellt, daß man in den erfindungsgemäßen Lösungen einen Teil der längerkettigen lipophilen Alkohole auch durch Fettsäureester bzw. deren Derivate ersetzen kann, wobei als solche Triglyceride und gegebenenfalls äthoxylierte Partialglyceridgemische zur Anwendung gelangen, wobei die Säurekomponente gegebenenfalls zusätzliche Hydroxylgruppen enthalten kann in einer Kettenlänge von $C_6$ bis $C_{21}$. Der Gehalt an diesen Fettsäureestern bzw. deren Derivate kann 1 bis 15 Vol.-% betragen.

Als Fettsäurester bzw. deren Derivate können z.B.

Kokosnußöl $(C_6 - C_{18})$

Palmöl $(C_{14} - C_{18})$

Ricinusöl $(C_{18})$

Rapsöl $(C_{14} - C_{18})$

bevorzugt äthoxyliertes Partialglyceridgemisch aus natürlichen gesättigten Pflanzenfettsäuren $(C_6 - C_{12})$

verwendet werden.

Unter kurzkettigen Alkoholen versteht man üblicherweise die unverzweigten, bzw. verzweigten Alkohole mit einer Kettenlänge von $C_1$ bis $C_5$, wobei im vorliegenden Fall Äthanol, Isopropanol und ein Gemisch aus beiden bevorzugt wird. Der Alkohol soll in Konzentrationen von 35 bis 98 Vol.-% eingesetzt werden.

Entschließt man sich für einen Zusatz von Wasser, so sollte dieser 50 Vol.-% nicht überschreiten.

Den erfindungsgemäßen Zubereitungen können die üblichen ätherischen Öle zugesetzt werden, und zwar dann in Konzentrationen von 0,1 bis 1 Vol.-%. Entschließt man sich zu den höherviskosen gallertartigen, bzw. gelartigen Lösungen,so ist es notwendig, gelbildende Zusätze zu verwenden. Darunter bieten sich vorzugsweise Celluloseätherderivate an, wobei die Konzentration der gelbildenden Zusätze im üblichen Rahmen liegt, d.h. 0,1 bis 2 Vol.-%.

Die erfindungsgemäße Grundlage gestattet es, wie schon ausgeführt, verschiedene Wirkstoffe wie Virustatika, Antibiotika, Chemotherapeutika, Antimykotika, Antihistaminika, Vasokonstriktoren sowie Antiphlogistika einzusetzen, um die entsprechenden Virusinfektionen, bakteriellen Infektionen, Mykosen und Entzündungen zu bekämpfen. Der Einsatz der erfindunggemäßen galenischen Lösungen gestattes es, schlecht penetrierende Wirkstoffe in ihrer Wirksamkeit zu steigern. Die Kombination zwischen den erfindungsgemäßen Lösungen und Virustatika sind deshalb besonders bevorzugt.

In den erfindungsgemäßen Zubereitungen erwies sich die Zusammensetzung der Ölkomponente als wesentlicher Bestandteil für die erhöhte Freisetzungsrate. Es wurde festgestellt, daß zur Ausbildung eines stabilen Öl-

films auf der Haut, insbesondere, wenn es sich um eine nässende Haut handelt, die Fettalkoholkomponente (z.B. Ricinoleylalkohol) die wesentliche Komponente zur Ausbildung eines stabilen Ölfilms darstellt, unter der Voraussetzung, daß leicht verdunstbare Lösungsmittel als Träger eingesetzt werden. Den Effekt eines Ölpflasters kann man nicht erzielen durch Zusatz von echten Ölen (Fettsäureester) zum alkoholischen Lösungsmittel, wie dies z.B. in manchen Haarwässern der Fall ist. Vielmehr führt die Verdunstung des Alkohols in diesem Fall zur Ausbildung von Öltröpfchen, die insbesondere auf nässender Haut abperlen. Zu einer erhöhten Wirkstofffreisetzung kommt es nicht.

Zum Nachweis der Wirkungssteigerung durch die erfindungsgemäßen Zubereitungen wurden antivirale Substanzen ausgewählt. Bei diesen antiviral wirksamen Substanzen handelt es sich vorwiegend um Nucleosid- bzw. Nucleotidabkömmlinge (z.B. Arabinofuranosyladenin, Arabinofuranosyladenin-5'-monophosphat, Trifluorthymidin, Joddesoxyuridin, Äthyl-desoxyuridin, Bromvinyldesoxyuridin, Bromvinylarabinofuranosyluracil, Acycloguanosin) um 5'-Ester dieser Verbindungen (z.B. Trifluorthymidin-5'- butyrat, Arabinofuranosylthymin-5'-valerat, Joddesoxyuridin-5'-pivalat) und um Phosphonoacylverbindungen (z.B. Phosphonoameisensäure, Phosphonessigsäure, Phosphonoessigsäureäthylester). All diesen Wirkstoffen ist eine sehr schwach ausgeprägte bzw. fehlende Lipophilie gemeinsam. Dies führt zwangsläufig zu einer schlechten dermalen Penetration dieser Substanzen aus den üblichen galenischen Grundlagen (Salben, Cremes, Gele, wäßrige Lösungen) und somit zu einer unbefriedigenden oder schlechten antiviralen Wirksamkeit dieser Stoffe bei dermatologischer Anwendung (Literatur K.K. Gauri Augenspiegel März 1980, 107-123 Stand der antiherpetischen Chemotherapie unter besonderer Berücksichtigung der Resistenz-Entwicklung.

Die Wirksamkeit der erfindungsgemäßen Arzneimittel wurde am Rücken von Meerschweinchen geprüft, die mit Herpes-simplex-Virus-1 infiziert wurden. Diese Versuche wurden im Vergleich zu bisher bekannten Standardzubereitungen wie Creme, Gel sowie alkoholischen Tinkturen unter Propandiolzusatz geprüft. Die Durchführung der Versuche erfolgt nach Angaben von Aleni und Oberg (Comparison of the therapeutic effect of 5 antiviral agents on cutanous herpes virus infection in guinea-pigs, Archives of virology 58, 277-288 (1978)). Die Ergebnisse der vergleichenden Untersuchungen sind in Tabelle 1 zusammengestellt. Es zeigte sich, daß die therapeutische Wirksamkeit der erfindungsgemäßen Zubereitungen wesentlich gesteigert ist im Vergleich zu den bekannten Zubereitungen. So werden antivirale Substanzen, die in Form von Gelen, Cremes, Salben oder herkömmlichen Tinkturen bei dermatologischer Anwendung nahezu unwirksam sind, durch Einarbeiten in die erfindungsgemäßen galenischen Zubereitungen gut bis sehr gut wirksam.

Dies zeigt eindeutig, daß nur durch die erfindungsgemäßen Zubereitungen die Bioverfügbarkeit der Wirksubstanzen so gesteigert werden kann, daß eine nachweisbare antivirale Wirksamkeit die Folge ist.

Rezepturbeispiele

1) Arabinofuranosyladenin-monophosphat-Tinktur (Spray)

1.a    2 g Arabinofuranosyladenin-monophosphat-
          Monohydrat 0,55 ml   10 nNaOH 40 ml   dest. Wasser 5 ml   Ricinoleylalkohol 5 ml   äthoxyliertes Partialglyceridgemisch.

       ad 100 ml mit Isopropanol auffüllen.

1.b    5 g Arabinofuranosyladenin-monophosphat-
          Monohydrat 2,7 ml    10 nNaOH 45  ml    dest. Wasser 3  ml    Ricinoleylalkohol 3  ml    äthoxyliertes Partialglyceridgemisch ad  100 ml mit Isopropanol auffüllen.

1.c    3 g Arabinofuranosyladenin- monophosphat-Monohydrat

       0,9  ml   10 nNaOH 40   ml   dest. Wasser 5   ml   Ricinoleylalkohol 3   ml   äthoxyliertes Partialglyceridgemisch ad   100 ml mit Äthanol auffüllen.

2. Bromvinylarabinofuranosyluracil-Tinktur (Spray)

       2 g Bromvinylarabinofuranosyluracil 20 ml Ricinoleylalkohol ad 100 ml mit 90%igem Äthanol auffüllen.

3. Bromvinylarabinofuranosyluracil-5'-monophosphat-
Tinktur (Spray)

3 g Bromvinylarabinofuranosyluracil-5'-monophosphat-Na-Salz

40 ml dest. Wasser 3 ml Ricinoleylalkohol ad 100 ml mit Isopropanol auffüllen.

4. Trifluorthymidin-Tinktur (Spray)

2,5 g Trifluorthymidin 20 ml Ricinoleylalkohol ad 100 ml mit Äthanol auffüllen.

5. Äthyldesoxyuridin-Tinktur (Spray)

2 g Äthyldesoxyuridin 10 ml Ricinoleylalkohol 1 ml äthoxyliertes Partialglyceridgemisch ad 100 ml mit 90%igem Äthanol auffüllen.

6. Fluoruracil-Tinktur (Spray)

1 g Fluoruracil 15 ml Ricinoleylalkohol ad 100 ml mit Äthanol/Wasser = 85:25 auffüllen.

7. Arabinofuranosylthymin-5'-valeriansäureester-
Tinktur (Spray)

2 g Arabinofuranosylthymin-5'-valerat 20 ml Ricinoleylalkohol ad 100 ml mit Äthanol auffüllen.

8. Phosphonoessigsäure-Tinktur (Spray)

1,5 g Phosphonessigsäure-Natriumsalz 40 ml dest. Wasser 3 ml Ricinoleylalkohol ad 100 ml mit Isopropanol auffüllen.

9. Phosphonoessigsäureäthylester-Tinktur (Spray)

2,5 g Phosphonoessigsäureäthylester-Ammoniumsalz 30 ml dest. Wasser 5 ml Ricinoleylalkohol 3 ml äthoxyliertes Partialglyceridgemisch ad 100 ml mit Äthanol auffüllen.


10. Trifluorthymidin-Salicylsäure-Tinktur (Spray)

2 g Trifluorthymidin 1 g Salicylsäure 10 ml Ricinoleylalkohol ad 100 ml mit Äthanol auffüllen.


11. Arabinofuranosyladenin-monophosphat-Gel

3 g Arabinofuranosyladenin-monophosphat-
Natriumsalz 40 ml Wasser 5 ml äthoxyliertes Partialglyceridgemisch 5 ml Ricinoleylalkohol 1,1 g Methylcellulose ad 100 ml mit Isopropanol auffüllen.


12. Antimykotikum

1,0 g    1-Propyl-3,5-Diäthyl-6-chloruracil 28,25 g   Isopropanol rein 0,75  g   Salizylsäure 20,0  g   Corol (Ricinoleylalkohol)


Alle anderen aufgeführten Beispiele sind Virustatika.

Tabelle 1 a

Testung von Ara-AMP (Arabinofuranosyladenin-5'-
monophosphat) in verschiedenen galenischen Zubereitungen auf antivirale Wirksamkeit am Meerschweinchenrücken.

| Zubereitung | Wirksamkeit |
|---|---|
| 1. Gel <br> Ara-AMP 3,0 g <br><br> Methylpropylzellulose 2,0 g <br><br> Propandiol 5 %  5,0 g <br><br> Aqua dest. ad. 100 g | 0 |
| 2. Creme <br> Ara-AMP 3,0 g <br> Cetylstearylalkohol DAB7   10,0 g <br> Emulgin B1                            3,0 g <br> Ölsäureoleylester DAB7        5,0 g <br> Paraffin subl.                    5,0 g <br> NaOH 5 N                          2,6 ml <br> dest. $H_2O$             ad 100,0 g | 0 |

| Zubereitung | Wirksamkeit |
|---|---|
| 3. **Tinktur**<br><br>Ara-AMP 3,0 g<br><br>äthoxyliertes Partialglyce-<br>ridgemisch 3 ml<br><br>Propandiol 50 ml<br><br>10 n NaOH 0,9 ml<br><br>Aqua dest. ad 100 ml | 1 |
| 4. **Erfindungsgemäße Zubereitung**<br><br>Ara-AMP 3 g<br><br>äthoxyliertes Partialglyce-<br>ridgemisch 3 ml<br><br>10 n NaOH 0,9 ml<br><br>Aqua dest. 40 ml<br><br>Ricinoleylalkohol 5 ml<br><br>Ethanol ad 100 ml | 4 |

Bewertung:   0 = unwirksam

                4 = maximale Wirksamkeit

<u>Tabelle 1 b</u>

Testung von BU-Ara-U (Bromvinylarabinofuranosyluracil) in verschiedenen galenischen Zubereitungen auf antivirale Wirksamkeit am Meerschweinchenrücken.

| Zubereitung | Wirksamkeit |
|---|---|
| 1. <u>Gel</u><br><br>BU-Ara-U 2,0 g<br>Methylpropylzellulose 2,0 g<br>Propandiol 5 % 5,0 g<br>Aqua dest. ad 100 g | 0 |
| 2. <u>Erfindungsgemäße Zubereitung</u><br>(Spray)<br>BU-Ara-U 2,0 g<br>Rizinoleylalkohol 20 ml<br>Ethanol 90% ad 100 ml | 4 |

Bewertung:   0 = unwirksam

             4 = maximale Wirksamkeit

DERMATOLOGISCHE ZUBEREITUNG

Patentansprüche

1. Topisch anzuwendende galenische dünnflüssige bis gallertartige klare Lösungen auf der Basis von kurzkettigen Alkoholen und gegebenenfalls Wasser für die dermatologische Anwendung, dadurch g e k e n n - z e i c h n e t , daß sie längerkettige, lipophile Alkohole und gegebenenfalls Fettsäureester, bzw. deren Derivate, enthalten.

2. Galenische Lösungen nach Anspruch 1, dadurch g e k e n n z e i c h n e t , daß sie als längerkettige lipophile Alkohole gesättigte und/oder ungesättigte Fettalkohole mit einer Kettenlänge von $C_{12}$ bis $C_{21}$ enthalten.

3. Galenische Lösungen nach Anspruch 1 oder 2, dadurch g e k e n n z e i c h n e t , daß diese Fettalkohole in Konzentrationen von 1 % bis etwa 50 % vorliegen.

4. Galenische Lösungen nach Anspruch 1 bis 3, dadurch g e k e n n z e i c h n e t , daß es sich bei den Fettsäureestern und deren Derivate um Triglyceride und gegebenenfalls äthoxylierte Partialglyceridgemische handelt, wobei die Säurekomponente gegebenenfalls zusätzliche Hydroxylgruppen enthalten kann, in einer Kettenlänge von $C_6$ bis $C_{21}$ vorliegen kann.

5. Galenische Lösungen nach Anspruch 4, dadurch g e k e n n z e i c h n e t , daß die Fettsäureester und deren Derivate in Konzentrationen von 1 % bis etwa 15 % vorliegen.

6. Galenische Lösungen nach einem der Ansprüche 1 bis 5, dadurch g e k e n n z e i c h n e t , daß es sich bei den kurzkettigen Alkoholen um Äthanol, Isopropanol oder einem Gemisch aus beiden handelt.

7. Galenische Lösungen nach Anspruch 6, dadurch g e k e n n z e i c h n e t , daß dieser Alkohol in Konzentrationen von 35 bis 98 Vol.-% vorliegt.

8. Galenische Lösungen nach einem der Ansprüche 1 bis 7, dadurch g e k e n n z e i c h n e t , daß Wasser in Konzentrationen bis zu 50 Vol.-% vorhanden ist.

9. Galenische Lösungen nach einem der Ansprüche 1 bis 8, dadurch g e k e n n z e i c h n e t , daß ätherische Öle in Konzentrationen von 0,1 bis 1 % vorliegen.

10. Galenische Lösugen nach Anspruch 1 bis 9, dadurch g e k e n n z e i c h n e t , daß die Lösungen gelbildende Zusätze enthalten.

11. Galenische Lösungen nach Anspruch 10, dadurch g e k e n n z e i c h n e t , daß es sich bei den gelbildenden Zusätzen um Celluloseätherderivate in Konzentrationen von 0,1 bis 2 % handelt.

12. Galenische Lösungen nach einem der Ansprüche 1 bis 11, dadurch g e k e n n z e i c h n e t , daß sie Wirkstoffe wie Virustatika, Antibiotika, Chemotherapeutika, Antimykotika, Antihistaminika, Vasokonstriktoren sowie Antiphlogistika enthalten.